(19) Europäisches Patentamt
European Patent Office
Office européen des brevets

(11) Publication number: **0 290 169 A2**

# (12) EUROPEAN PATENT APPLICATION

(21) Application number: 88303616.2

(51) Int. Cl.4: C07F 15/00 , A61K 31/28

(22) Date of filing: 21.04.88

(30) Priority: 04.05.87 US 46476

(43) Date of publication of application: 09.11.88 Bulletin 88/45

(84) Designated Contracting States: AT BE CH DE ES FR GB GR IT LI LU NL SE

(71) Applicant: ENGELHARD CORPORATION
Menlo Park, CN 40
Edison New Jersey 08818(US)

(72) Inventor: Hollis, Leslie Steven
33, Guilford Lane
Mercerville New Jersey 08619(US)
Inventor: Amundsen,Alan R.
12 Meadowbrook Drive
Somerville New Jersey 08876(US)
Inventor: Stern, Eric W.
234 Oak Tree Road
Mountainside New Jersey 07092(US)
Inventor: Miller, Arthur V.,III
9 Cherrytree Circle
Howell New Jersey 07731(US)

(74) Representative: Fisher, Adrian John et al
CARPMAELS & RANSFORD 43 Bloomsbury Square
London WC1A 2RA(GB)

## (54) Diamineplatinum complexes with phosphonocarboxylate and substituted phosphonocarboxylate ligands as antitumor agents.

(57) Novel diamineplatinum complexes with phosphonocarboxylate and substituted phosphonocarboxylate ligands are disclosed. These complexes exhibit an antitumor effect and are characterized by low mammalian toxicity.

EP 0 290 169 A2

# DIAMINEPLATINUM COMPLEXES WITH PHOSPHONOCARBOXYLATE AND SUBSTITUTED PHOSPHONOCARBOXYLATE LIGANDS AS ANTITUMOR AGENTS

## Background of the Invention

In recent years cisplatin, cis-[$Pt(NH_3)_2Cl_2$], has achieved success as a chemical mediator of neoplastic disease. It has, for example, been successful in bringing about regression of testicular, ovarian and bladder carcinomas. While it has demonstrated success in these areas, its inability to demonstrate useful activity in treating such major forms of the disease as breast, lung and colon cancer has stimulated the search for new platinum based antitumor agents. Research in this area has produced a number of promising new compounds that have shown good activity in various animal tumor screens. See, for example, Prestayko, A.E. et al, "Cisplatin, Status and New Developments" Academic Press: New York, 1980.

A number of these compounds are being evaluated in the clinic with the hope of developing an effective platinum based antitumor agent that has a significantly different spectrum of activity and improved toxicological properties. Also of importance are compounds with superior physical and chemical properties such as aqueous solubility and stability greater than cisplatin.

The present invention is directed to a series of platinum compounds, diamineplatinum(II)-phosphonocarboxylates and substituted phosphonocarboxylates, which show excellent antitumor activity and both high water solubility and solution stability. It is also directed to novel compositions containing such compounds and a method for the treatment of malignant tumor cells which are sensitive to the compounds via the administration of the composition.

Some platinum phosphonocarboxylate compounds have been reported in the prior art. For example, U.S. Patent Nos. 4,562,275 and 4,594,418 disclose platinum(II) complexes of 1,2-diaminocyclohexane, l-aminomethylcyclooctylamine and 1,2-diaminodimethylpentane. Specific examples describe two compounds with phosphonoacetate ligands, [Pt(1,2-diaminocyclohexane)-($O_2CCH_2PO_3H$)] and [Pt(1-aminomethylcyclooctylamine)-($O_2CCH_2PO_3H$)]. These compounds are different than the compounds of the present invention. Moreover, the method of preparation described for these compounds produces products which are less pure than the methods described herein.

## SUMMARY OF THE INVENTION

The platinum compounds of this invention are a new series of diamineplatinum(II) complexes containing a variety of phosphonocarboxylate ligands. These compounds are novel in that they are anionic at neutral pH; typically, platinum-based antitumor agents are neutral species in solution. The compounds exhibit excellent activity against malignant tumors in animals and low mammalian toxicity. At least one compound in this series was found to be less kidney toxic than cisplatin. In addition, the compounds have good solubility and stability in water.

Detailed Description of the Invention

The present invention is directed to compounds of the following general formula:

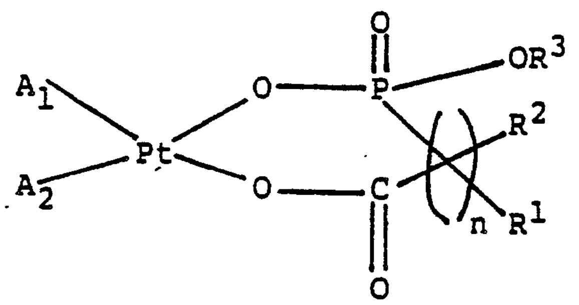

(I)

wherein $A_1$ and $A_2$ are ammonia or aliphatic amine ligands or $A_1$ and $A_2$, taken together, are a chelating diamine ligand.

The monodentate amine ligands $A_1$ and $A_2$ may be the same or different and can be represented by the formula $RNH_2$ and include ammonia ($NH_3$) and monoalkyl amines having up to 6 carbons in the alkyl group as, for example, methylamine, ethylamine, propylamine, isopropylamine, hexylamine and the like. Alkyl amines having up to 3 carbon atoms are presently preferred. The alkyl group, R, also may contain substituents such as hydroxy, $C_1$-$C_4$ alkoxy, halo, carboxy, $C_1$-$C_4$ alkylcarboxy, or $C_1$-$C_4$ alkoxycarbonyl.

The chelating bidentate diamine ligands represented by $A_1$ and $A_2$, taken together, are represented by the following formula (II):

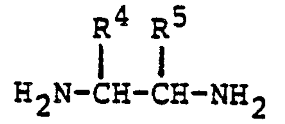

(II)

wherein each of $R^4$ and $R^5$ taken separately, represent hydrogen or lower alkyl and $R^4$ and $R^5$, taken together, represent a 1,2-cycloalkyl containing from about 4-8 nuclear carbon atoms affording, for example, a l,2-diaminocyclopentane ligand ,1,2-diaminocyclohexane ligand and l,2-diaminocyclheptane ligand. The cycloalkyl may optionally be substituted on the nuclear carbons by one or more linear or branched chain lower alkyl groups, hydroxy, $C_1$-$C_4$ alkoxy, halo, carboxy, $C_1$-$C_4$ alkylcarboxy or $C_1$-$C_4$ alkoxycarbonyl. As used herein, the term "lower alkyl" means a linear or branched chain alkyl group of methyl, ethyl, or propyl. Preferred diamine ligands (II) are those in which at least one of the $R^4$ and $R^5$ radicals represents hydrogen as, for example, ethylenediamine and propylene-1,2-diamine. Also preferred are diamines wherein $R^4$ and $R^5$, taken together, form a 1,2-diaminocyclohexane moiety. $R^4$ and $R^5$, taken separately, also may represent such groups as hydroxy, $C_1$-$C_4$ alkoxy, halo, carboxy, $C_1$-$C_4$ alkylcarboxy, $C_1$-$C_4$ alkoxycarbonyl or a linear or branched lower alkyl substituted with any of hydroxy, $C_1$-$C_4$ alkoxy, halo, carboxy, $C_1$-$C_4$ alkylcarboxy or $C_1$-$C_4$ alkoxycarbonyl.

The phosphonocarboxylate ligand may be phosphonoformate (n=O), phosphonoacetate (n=1) or phosphonopropionate (n=2). Each of the phosphonoacetate and phosphonopropionate (when n >0) ligands may be substituted by $R^1$ and/or $R^2$ at the positions shown in the general formula I. $R^1$ and $R^2$ may be the same or different and are selected from the group consisting of hydrogen; OH; linear or branched $C_1$-$C_{16}$ alkyl; linear or branched $C_1$-$C_{16}$ alkyl ether; linear or branched $C_1$-$C_{16}$ primary or secondary alkyl amine in which the alkyl group may contain substituent functional groups such as hydroxyl, halo, carboxylic acid, carboxylic acid halide, carboxylic acid hydrazide, ester, primary or secondary amide, carboxylic acid anhydride, aldehyde, acetal, hemiacetal, ketone, ketal, -O-acylurea, imidazole, methoxymethyl ester, tetrahydropyranyl ester, a sulfonic acid ester, such as tosyl, mesyl or brosyl, hydrazine, hydrazone, semicarbazone, phenyl or substituted phenyl wherein the phenyl may be substituted with any of the substituent functional groups; linear or branched $C_1$-$C_{16}$ alkyl ether substituted by a substituent functional group as described above in this paragraph; $C_1$-$C_{16}$ linear or branched alkenyl in which the alkenyl group may contain the same substituent functional groups as described above in this paragraph; linear or branched substituted $C_1$-$C_{16}$ alkyl in which the alkyl contains substituent functional groups as described

above in this paragraph; phenyl in which the phenyl group may contain substituent functional groups as described above or $C_1$-$C_{16}$ alkyl, or $C_1$-$C_{16}$ alkenyl groups or substituted alkyl or alkenyl substituted with the substituent functional groups as described above in this paragraph; and benzyl which may contain substituent functional groups as described above in this paragraph or $C_1$-$C_{16}$ alkyl or $C_1$-$C_{16}$ alkenyl groups or substituted alkyl or alkenyl groups as described above in this paragraph.

The $R^3$ group in formula (I) may be selected from alkali metal cations ($Li^+$, $Na^+$, $K^+$), $H^+$, or $C_1$-$C_4$ alkyl. The compounds may be used as such or may be linked to a monoclonal antibody via the substituent functional groups on $R^1$ and $R^2$ as described in the preceding paragraph.

## Pharmacology

The products of this invention are useful in the treatment of malignant tumor cells sensitive thereto in animals as, for example, Sarcoma 180 ascites and Ll210 leukemia in mammals such as mice. This antitumor cell effect also may extend to other sarcomas and leukemias and to such other tumor cells as lymphoid leukemia, lymphosarcoma, myelocytic leukemia, malignant lymphoma, squamous cell carcinoma, adenocarcinoma, scirrhous carcinoma, malignant melanoma, seminoma, teratoma, choriocarcinoma, embryonal carcinoma, cystadenocarcinoma, endometroidcarcinoma or neuroblastoma and the like. In addition, the complexes may be useful as anti-viral, anti-inflammatory, anti-bacterial and anti-parasitic agents.

The complexes of this invention may be administered parenterally or orally in a mixture with a non-toxic pharmacologically acceptable inert carrier or diluent in any of the usual pharmaceutical forms. These include solid and liquid oral unit dosage forms such as tablets, capsules, powders and suspensions or solutions and suspensions for subcutaneous, intramuscular, intravenous, or intraarterial injection.

The term "unit dosage" refers to physically discrete units which may be administered in a single or multiple dosages each containing a predetermined quantity of active ingredient in association with the required diluent, carrier or vehicle. The quantity of active ingredient is the amount of the complex which is needed to produce the desired therapeutic effect.

A typical unit dosage consists essentially of from about 10 to 450 mg of active ingredient; however, the form in which the ingredient is administered and the frequency of administration in usually determinative of the concentration. Thus, for example, oral unit dosage forms containing 20 to 450 mg of active ingredient may be administered one or more times per day depending upon the severity of the tumor cells which is sought to be treated and the condition of the host animal. By contrast, parenteral administration generally requires from about 10 to about 100 mg of the active ingredient per unit dosage administered as a daily dose or as a fraction thereof depending upon whether the regimen calls for administration once, twice, three or four times daily.

By contrast to the unit dosage, the effective dose is that dosage which is needed to achieve the desired anti-tumor effect. In general, this dosage lies within the range of from about 10 to 950 mg of the active ingredient per kg of body weight of the host animal. A preferred concentration lies within the range of from about 30 to 450 mg/kg of body weight. For oral administration it has been found that an effective dose of about 50 to 950 mg/kg is most suitable, whereas in the case of parenteral administration it is usual advisable to employ from about 30 to 350 mg/kg. These unit dosages are well below the toxic or lethal dose and they may be varied over a wide range for adjustment to the patient who is being treated.

According to the present invention, the term "pharmacologically acceptable inert carrier or diluent" means a non-toxic substance which, when mixed with the active ingredient, renders it more suitable for administration. Compositions intended for oral administration may include such carriers or diluents as corn starch, potato starch, sodium carboxymethyl cellulose, ethyl cellulose, cellulose acetate, powder gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and dicalciumphosphate. The compositions may also contain non-toxic adjuvants and modifiers such as dyes, buffering agents, preservatives, surfactants, emulsifiers, flavoring agents, biocides and the like.

Tablets are prepared by mixing a complex of this invention in a suitably comminuted or powdered form with a diluent or base such as starch, kaolin, dicalciumphosphate and the like. The resulting mixture can be granulated by wetting with a binder such as a syrup, starch (paste), acacia mucilage or solutions of cellulosic or polymeric materials, whereafter, the wetted mixture is formed through a screen. As an alternative to granulating, the powder mixture can be run through a tablet machine and any imperfectly formed slugs broken into granules. The granules are lubricated to prevent sticking to the tablet-forming dyes via the addition of stearic acid, a stearate salt, talc or mineral oil and the lubricated mixture is then

compressed into tablets. The complexes can also be combined with free flowing inert carriers followed by compression into tablets without going through the granulated or slugging steps. A protective coating or sealing coat of shellac, sugar or polymeric material and a polished coating of wax can also be provided. Dye stuffs may be added to distinguish different unit dosages.

Dry or hard filled capsules are formulated by preparing a powdered mixture, according to the procedure herein before described and pouring the mixture into preformed gelatin sheets. A lubricant such as talc, magnesium stearate or calcium stearate can be added prior to the filling operation. A glidant such as coloidal silica may be added to improve the flow characteristics and a disintegrating or solubilizing agent may also be added to enhance the effectiveness of the medicament upon ingestion.

In soft gelatin capsules, the active complex is dissolved or suspended in vegetable oil, peanut oil, alcohol or glycerine and the like.

Powders for addition to foods, drinking water, fruit juice or other potable liquids are prepared by comminuting the compound to a fine size and mixing with a similarly comminuted pharmaceutical diluent or carrier such as an edible carbohydrate as, for example, starch. Sweetening agents and flavorings, preservatives and dispersing and/or coloring agents may also be employed.

Oral fluids such as syrups and elixirs are prepared in unit dosage forms so that a given quantity of medicament, such as a teaspoonful, will contain a predetermined amount of the active ingredient. Suspensions can be formulated by dispersing the active ingredient in a non-toxic vehicle in which it is essentially insoluble.

Compositions intended for parenteral administration may include such diluents and carriers as water or water-miscible solvents as, for example, sesame oil, groundnut oil, aqueous propylene glycol and a solution of sodium riboflavin. Typical of said compositions are solutions which contain the active ingredient in sterile form. Alternatively, a unit dosage form for parenteral administration can be prepared by placing a measured amount of complex in solution in sterile form, removing the solvent by liophylization and sealing the vial. An accompanying vial of sterile vehicle can be provided for mixing with the complex prior to administration.

According to the present invention two or more of the complexes may be combined into a single unit dosage form or, alternatively, one or more of the complexes of the present invention may be combined with other known anti-tumor agents, therapeutic agents or nutrative agents so as to enhance or compliment the antitumor effect.

The preferred compositions for oral administration are tablets in which the complexes of the present invention are present in quantities of about 5 to about 375 mg but, preferably, about 10 to 200 mg in a pharmaceutically acceptable orally ingestible solid carrier. If desired, the compositions may also contain flavorants, binders, lubricants and other excipients known in the art.

A preferred alternative for oral administration is the soft gelatin capsule. Such a composition may contain from about 5 to about 375 mg, but, preferably, about 10 to 200 mg by weight of active ingredient dissolved or suspended in vegetable oil, peanut oil, alcohol or glycerin and the like.

A preferred unit dosage form for parenteral administration will contain complexes of the present invention of about 5 to 100 mg, but preferably 5 to 50 mg.

The following embodiments illustrate representative unit dosage forms:

Compressed Tablet

| | |
|---|---|
| Na[Pt(trans-R,R-dach)(2-phosphonovalerate)] | 200 mg. |
| Niacinamide | 50 mg. |
| Calcium Pantothenate | 20 mg. |
| Magnesium Sulfate | 50 mg. |
| Zinc Sulfate | 50 mg. |
| Magnesium Stearate | 10 mg. |
| | 380 mg. |

The 2-phosphonovalerate complex, niacinamide, calcium pantothenate, magnesium sulfate, zinc sulfate and magnesium stearate (5.0 mg.) are mixed and compressed into slugs. The slugs are then broken into granules and sifted through an 8 mesh screen. Additional magnesium stearate (5.0 mg.) is added and the mixture is then compressed into tablets suitable for oral administration.

Soft Gelatin Capsule

A soft elastic gelatin capsule is filled with the following ingredients:

| | |
|---|---|
| Na[Pt(trans-R,R-dach)(2-methyl-2-phosphonobutyrate)] | 100 mg. |
| Wheat germ oil | 50 mg. |
| Sunflower seed oil | 100 mg. |
| | 250 mg. |

The 2-methyl-2-phosphonobutyrate complex and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

Dry Filled Capsule

A hard dry-filled capsule may be prepared from the following ingredients:

| | |
|---|---|
| Na[Pt(1,2-propylenediamine)(2-phosphonobutyrate)] | 200 mg. |
| Niacinamide | 50 mg. |
| Calcium Pantothenate | 10 mg. |
| Sodium Ascorbate | 150 mg. |
| | 410 mg. |

The 2-phosphonobutyrate complex is reduced to a No. 60 powder. Niacinamide, calcium pantothenate and sodium ascorbate are passed through a No. 60 bolting cloth and these ingredients are added to the propylenediamineplatinum 2-phosphonobutyrate complex. This combination of ingredients is mixed for 10 minutes and then poured into a No. 3 size gelatin capsule.

Dry Powder

The following composition illustrates a representative dosage in dry powder form. In this embodiment the active ingredient is water soluble and it is combined with up to 60% by weight of a suitable flavoring agent. All quantities are in a weight-percent relationship.

| | |
|---|---|
| Na[Pt(trans-R,R-dach)(2-phosphono-1,7-heptanedicarboxylate) | 25-90% |
| Flavoring Agent | 10-60% |
| Preservative | 0.1% |

Parenteral Solution

Injectable solutions can be formulated by mixing an ampoule of active ingredient with an ampoule of sterile diluent:

Ampoule: Na[Pt(trans-R,R-dach)(2-phosphonophenylacetate)] 100 mg.

Ampoule: Sterile water (Diluent for Injection) 4 cc.

The 2-phosphonophenylacetate complex and water are mixed thoroughly immediately prior to administration. If desired, one or more other active ingredients may be added to provide an injectable solution having enhanced therapeutic activity.

## Preparative Methods

The platinum(II) phosphonocarboxylate complexes of this invention are prepared by reacting a diamineplatinum(II) dihalide with, preferably, one equivalent of $Ag_2SO_4$ or two equivalents of $AgNO_3$. The reaction is carried out in the dark at temperatures from ambient to 80°C, preferably at ambient to 50°C for a period from several hours to 36 hours. The resulting insoluble silver halide is removed by filtration. The aquated diamineplatinum(II) nitrate is reacted in water with one equivalent of phosphonocarboxylate (as free acid) and 3 equivalents of alkali hydroxide. After stirring for 2-24 hours at 20-30°C., the solution is evaporated to dryness and the resulting product is purified by recrystallization or reprecipitation from water/alcohol (methanol or isopropanol) or water/acetone mixtures.

In the case of the diamineplatinum(II) aquasulfate, one equivalent of $Ba(OH)_2$ and one equivalent of alkali metal hydroxide are added. The solution is then stirred at 20-30°C. for up to 24 hours and the $BaSO_4$ is removed by filtration. The filtrate is evaporated to dryness and the product is purified as described above.

Examples 1-23, illustrate the methods by which the compounds of this invention may be prepared. Example 24 describes the protocol used to evaluate their efficacy in mice. These, examples are illustrative only and the invention should not be construed as being limited thereto because it will be apparent to one of ordinary skill that obvious modifications may be effected and functionally equivalent reagents may be substituted for those recited without departing from the spirit or scope of this invention.

## EXAMPLE 1

### Platinum Amine Sulfates

In the present invention, the diamineplatinum(II) sulfate starting materials are prepared by reacting the corresponding diaminediiodoplatinum(II) complex with silver sulfate in water. The diaminediiodide complexes were prepared by reacting the appropriate amine or diamine with $K_2PtI_4$, using the method of Dhara as described in Dhara, S.G.; "Indian J. Chem.", 1970, 8, 193 which is incorporated herein by reference.

The following general procedure given for preparation of $Pt(en)(SO_4)(H_2O)$ (en = ethylenediamine) was used for preparation of $Pt(diamine)(SO_4)(H_2O)$ in other Examples employing these materials.

A suspension of $Pt(en)I_2$ (50 mmole, 25.45 g) in 500 mL of water was stirred with $Ag_2SO_4$ (50 mmole, 15.52 g) for 36 hours in the dark. The resulting AgI precipitate was removed by filtration and the filtrate evaporated to dryness on a roto-evaporator. The resulting light yellow solid was dried for 48 hours under vacuum (yield 16.2 g). A platinum analysis, by the ash method, suggests that the product is best formulated as $Pt(en)(SO_4)(H_2O)$.

### Phosphonocarboxylate Ligands

The various phosphonocarboxylates used in the preparation of Pt complexes described in the Examples were either purchased from commercial sources including Alfa-Ventron, Fairfield Chemical and Sigma Chemical as free acids or as triethyl esters from which the corresponding free acids were prepared as described in Example 2 for 2-phosphonovaleric acid. Some mono and disubstituted phosphonocarboxylates were prepared by alkylation (Examples 3-5) employing the method described by G.M. Kosolapoff and J.S. Powell in J.Am Chem. Soc., 1950, 72, 4189, which is incorporated herein by reference.

EXAMPLE 2

2-Phosphonovaleric Acid

A solution of 50 g of triethyl-2-phosphonovalerate in 250 mL of aqueous HBr (48%) was heated to 80°C for 48 hours. The resulting amber solution was evaporated in the fume hood over a period of four days. The resulting oil was dissolved in acetone and filtered through charcoal and the filtrate was re-evaporated in the hood. Crystals of the free acid were collected, washed with toluene and dried under vacuum. The product was found to be pure by $^{13}C$ and $^{31}P$ NMR.

EXAMPLE 3

2-Methyl-2-phosphonobutyric Acid

A solution of 25 g of triethyl-2-phosphonobutyrate in 200 mL of xylene was treated with 4.46 g of K metal and heated to reflux for one hour. The resulting potassium salt of the 2-phosphonobutyrate ester was treated with 16 mL of ethyl iodide (by slow addition). This mixture was refluxed for 2 hours, followed by 18 hours of stirring at room temperature. After removing the KI by filtration and the xylene/EtI by roto-evaporation, the product was collected by vacuum distillation (bp 105° at ~ 0.1 mm Hg). Yield of triethyl-2-methyl-2-phosphonobutyrate was 20 g (71%). The free acid was obtained by hydrolyzing the ester as described in Example 2. The product identity was confirmed by $^{13}C$ and $^{31}P$ NMR.

EXAMPLE 4

2-phosphono-1,7-heptanedicarboxylic Acid

A solution of 25 g of triethylphosphonoacetate in 200 mL of xylene was treated with 4.53 g of K metal and the resulting solution was refluxed for 1 hour. Ethyl-5-bromovalerate (19.4 mL) was slowly added to this solution and the mixture was refluxed for 3 hours, followed by stirring at room temperature for 10 hours. The KBr was filtered and the xylene removed by roto-evaporation. The product was collected by vacuum distillation (bp 165° at ~ 0.1 mm Hg). The resulting triethyl ester was hydrolyzed using aqueous HBr as described in Example 2. Approximately 10 g of free acid (light yellow oil) was obtained. The product appeared pure as judged by $^{13}C$ and $^{31}P$ NMR.

EXAMPLE 5

2-(2-hydroxyethyl)phosphonoacetic Acid

A solution of 5 g of diethylphosphonobutyrolactone in 15 mL of aqueous HBr (conc.) was refluxed for 3 hours. The resulting solution was evaporated under N2 over a period of 3 days. The oily product was dissolved in acetone and treated with concentrated $NH_4OH$ solution. The resulting precipitate was collected, washed with methanol and dried under vacuum. The $NH_4^+$ salt was redissolved in water and treated with HCl (to pH 1) and roto-evaporated to dryness. The free acid was identified using $^{31}P$ and $^{13}C$ NMR.

EXAMPLE 6

Na[Pt(trans-R,R-dach)(2-phosphonophenylacetate)]

A solution of Pt(trans-R,R-dach)($SO_4$)($H_2O$) (3.314 mmole) (dach = 1,2-diaminocyclohexane) and 2-phosphonophenylacetic acid (3.314 mmole) in 100 mL of water was mixed with 3.314 mmole of Ba(OH)-$_2$•8$H_2O$ and 3.314 mmole of NaOH. After 24 hours of stirring at room temperature, the resulting $BaSO_4$ precipitate was removed by filtration. The yellow filtrate was roto-evaporated to dryness at 50°C, yielding a grey-white solid. The solid was stirred in isopropanol, filtered and dried under vacuum for 24 hours. The dried product was redissolved in methanol (100 mL) and filtered to remove a small amount of insoluble material. Upon evaporation of the methanol filtrate, 0.35 g of purified Na[Pt(trans-R,R-dach)(2-phosphonophenylacetate)] was obtained. NMR data obtained on this material are presented in Tables 1 and 2.

EXAMPLE 7

Na[cis-Pt($NH_3$)$_2$(2-phosphonophenylacetate)]

A solution of 11.7 mmole of cis-Pt($NH_3$)$_2$($SO_4$)($H_2O$) and 11.7 mmole 2-phenylphosphonoacetic acid in 125 mL of water was treated with 11.7 mmole Ba(OH)$_2$•8H20 and 11.7 mmole NaOH. After 24 hours, the $BaSO_4$ precipitate was removed by filtration and the resulting product was worked up from the filtrate as described in Example 6 above (yield 0.9 g of a yellow-white crystalline product). NMR data obtained on this sample are given in Tables 1 and 2.

EXAMPLE 8

Na[Pt(pn)(2-phosphonobutyrate)]

The l,2-propylenediamine (pn) complex was prepared by reacting Pt(pn)($SO_4$)($H_2O$) (10 mmole) with 10 mmole of 2-phosphonobutyric acid, 10 mmole of Ba(OH)$_2$•8$H_2O$ and 10 mmole NaOH in 200 mL of water. After 24 hours at room temperature, the $BaSO_4$ was removed by filtration and the filtrate was roto-evaporated to dryness at 55°C. The resulting solid was suspended in isopropanol, filtered and dried under vacuum for 48 hours. A yield of 3.55 g of a yellow-white crystalline solid was obtained. NMR data for this sample are given in Tables 1 and 2. Elemental analysis was performed and is summarized in Table 3.

EXAMPLE 9

Na[cis-Pt(i-PrNH$_2$)$_2$(phosphonoacetate)]

Method 1

A solution of Pt(isopropylamine)$_2$($SO_4$)($H_2O$) (20 mmole) phosphonoacetic acid (20 mmole) and Ba(OH)-$_2$•8$H_2O$ (20 mmole) in 200 mL of water was stirred for 30 minutes at room temperature. Sodium hydroxide (20 mmole) was then added to the solution and after 24 hours, the $BaSO_4$ precipitate was removed by filtration. The filtrate was roto-evaporated to dryness and the resulting solid was suspended in isopropanol (100 mL) and filtered. A yield of 9.8 g of white solid was obtained after vacuum drying. NMR data are given in Tables 1 and 2 and the elemental analysis is summarized in Table 3.

Method 2

This method uses silver nitrate to prepare the diaqua diamineplatinum(II) nitrate intermediate which is then reacted with the phosphonoacetic acid ligand. While this method does produce the desired material, the $BaSO_4$ precipitation (Method 1) gives a salt-free product that is easier to purify.

A suspension of cis-Pt(i-$PrNH_2)_2I_2$ (10 mmole) and $AgNO_3$ (20 mmole) in 55 mL of water was stirred for 45 minutes at 45-50°C. After cooling to 25°C, the AgI precipitate was removed by filtration. A solution of phosphonoacetic acid (11 mmole) in 50 mL of water was adjusted to pH 6.4 with 6 M NaOH and then added to the filtrate. After stirring for two hours at room temperature, the solution was roto-evaporated to dryness at 50°C. The resulting white solid was suspended in isopropanol and filtered, yielding 7.6 g of product after drying. The product was reprecipitated from water and isopropanol and re-dried under vacuum.

EXAMPLE 10

H[Pt(pn)(phosphonoacetate)]

Pt(pn)($SO_4$)($H_2O$) (20.0 mmole) (pn = l,2-propylenediamine), phosphonoacetic acid (20.0 mmole) and $Ba(OH)_2 \bullet 8H_2O$ (20.0 mmole) were dissolved in 200 mL of water and stirred for 18 hours at 25°C. The resulting $BaSO_4$ precipitate was removed by filtration and the filtrate evaporated to dryness under vacuum (yield 5.76 g).

EXAMPLE 11

Na[Pt(pn)(phosphonoacetate)]

A 2.0 g portion of the product in Example 10 was stirred in 5 mL water and adjusted to pH 4.0 with 2 N NaOH. The solution was filtered into absolute ethanol and the resulting precipitate was isolated, washed with methanol and dried. NMR data are given in Tables 1 and 2 and elemental analysis is tabulated in Table 3.

EXAMPLE 12

Na[Pt(pn)(2-phosphonopropionate)]

Pt(pn)($SO_4$)($H_2O$) (10 mmole), 2-phosphonopropionic acid (10 mmole) and barium hydroxide (10 mmole) were stirred in 200 mL $H_2O$ for 30 minutes. Sodium hydroxide (10 mmoles in 5 mL $H_2O$) was added to the solution and after 24 hours of stirring at room temperature, the $BaSO_4$ precipitate was filtered off. The filtrate was evaporated to dryness and the solid was suspended in 100 mL of isopropanol. After filtration the product was redissolved in 100 mL $H_2O$ and then taken to dryness and vacuum dried. Yield was 4.3 g of a yellow-white crystalline material. NMR data are given in Tables 1 and 2.

EXAMPLE 13

H[Pt(en)(phosphonoformate)]

$Pt(en)I_2$ (10 mmole) (en = ethylenediamine) and $AgNO_3$ (20 mmole) were stirred at 50°C in 80 mL $H_2O$ for one hour. After cooling the solution to 25°C, the resulting AgI precipitate was filtered off. To the remaining bright yellow solution (pH 2.28) was added trisodium phosphonoformate (10 mmole in 10 mL $H_2O$). The pH of the solution increased to 6.4, and after 30 minutes of stirring at room temperature, a yellow precipitate formed. The pH was readjusted to 5.0 with 0.1 N $HNO_3$ and the mixture was stirred for an additional 22 hours. A yellow precipitate was filtered off and the remaining solution was rotary evaporated and the residue redissolved in water (10 mL). After filtering, the solution was adjusted to pH 1.0 with concentrated $HNO_3$ and cooled to 5°C for one hour. The resulting yellow precipitate was removed by filtration and washed with water (3 mL) and two portions each of isopropanol (3 mL) and ethyl ether (3 mL). The yield of the dried product was 1.6 g. Aqueous solutions of this product were readjusted to pH 6.5 with 6 N NaOH for animal administration. NMR data are given in Tables 1 and 2.

EXAMPLE 14

Na[cis-$Pt(NH_3)_2$(2-phosphonobutyrate)]

A mixture of 10 mmole each of cis-$Pt(NH_3)_2(SO_4)(H_2O)$, $Ba(OH)_2 \bullet 8H_2O$ and 2-phosphonobutyric acid in 200 mL of water was stirred at room temperature. After ten minutes, 10 mmole of sodium hydroxide was added, and the solution was stirred for an additional 24 hours. The resulting barium sulfate precipitate was removed by filtration and the remaining filtrate was roto-evaporated to dryness. The solid was stirred in isopropanol, filtered and vacuum dried. A yield of 4.06 g of yellow crystalline product was obtained. Purification of the compound was accomplished by stirring 2 g of the solid in 5 mL water for 24 hours. Insoluble material was removed by filtration and the product was isolated by evaporating the filtrate (yield 1.5 g). NMR data are given in Tables 1 and 2.

EXAMPLE 15

Na[cis-$Pt(NH_3)_2$(2-phosphonopropionate)]

A solution of 15 mmole of cis-$Pt(NH_3)_2(SO_4)(H_2O)$ and 15 mmole $Ba(OH)_2 \bullet 8H20$ in 300 mL water was mixed at 50°C for 40 minutes. 2-phosphonopropionic acid (15 mmole) was added and the resulting mixture was stirred for an additional four hours. After removing the barium sulfate precipitate by filtration, the filtrate was roto-evaporated to 15 mL. The solution was cooled to 5°C for four hours which produced a green precipitate (1.1 g) that was filtered and washed with water and ethanol. Two additional crops were obtained in this manner. Crop 1 was dissolved in 10 mL water by adjusting the pH to 7 with NaOH. The solution was filtered and evaporated to dryness, and the solid was suspended in ethanol and isolated by filtration. This product was found to be pure using NMR (Tables 1 and 2).

EXAMPLE 16

H[Pt(en)(phosphonoacetate)]

$Pt(en)I_2$ (20 mmole) and silver nitrate (40 mmole) were stirred at 45°C in 150 mL water for one hour. The solution was cooled to 25°C and the resulting silver iodide precipitate was removed by filtration. Solutions of phosphonoacetic acid (21 mmole in 5 mL water) and sodium hydroxide (20 mmole in 5 mL water) were combined and then added to the filtrate. This solution was stirred at 25°C under nitrogen for

two hours and reduced to 5 mL of lime green oil by evaporation. The oil was stored in 100 mL absolute ethanol at 5°C for 24 hours producing a green precipitate (yield 8.39g) which was collected by filtration.

A portion of the product (1.5 g) was purified by heating the solid to 80°C in 5 mL of water. The pH of the mixture (initially 2.3) was adjusted with sodium hydroxide (6 N) until most of the solid dissolved (pH 4.3). Following filtration to remove insolubles, the filtrate was reacidified (pH 2.5) and the resulting precipitate was collected by filtration. The product was washed with methanol (5 mL) and vacuum dried. (Yield 0.88g).

## EXAMPLE 17

Na[Pt(en) (phosphonoacetate)]

A combination of 20.24 mmole each of $Pt(en)(SO_4)(H_2O)$, phosphonoacetic acid and $Ba(OH)_2{\bullet}8H_20$ was stirred at room temperature in 200 mL of water for 18 hours. The solution was reduced by evaporation to 50 mL and stored at 5°C for 24 hours. A greenish white precipitate was isolated from the solution by filtration. A 0.96 g portion of this solid was redissolved in 10 mL water and adjusted to pH 7 with 2 M NaOH. After filtration and evaporation the solid was stirred in ethanol, filtered and dried. Elemental analysis is shown in Table 3. NMR data are presented in Tables 1 and 2.

## EXAMPLE 18

Na[Pt(trans-R,R-dach)(2-phosphonovalerate)]

A mixture of 10 mmole each of Pt(trans-R,R-dach)$(S0_4)(H_20)$, $Ba(OH)_2{\bullet}8H20$, sodium hydroxide, and 2-phosphonovaleric acid was stirred at 25°C in 200 mL water for 24 hours. The remainder of the work-up was identical to that previously outlined for Na[cis-Pt$(NH_3)_2$(2-phosphonobutyrate)]; Example 14. The cream colored product was judged to be pure via NMR (see Tables 1 and 2).

## EXAMPLE 19

Na[cis-Pt$(NH_3)_2$(2-phosphonovalerate)]

A solution of 10 mmole each of cis-Pt$(NH_3)_2(SO_4)(H_2O)$, $Ba(OH)_2{\bullet}8H_20$ NaOH and phosphonovaleric acid, in 200 mL of water was stirred for 24 hours at room temperature. The reaction was worked-up in a manner analogous to that of the Na[cis-Pt$(NH_3)_2$(2-phosphonobutyrate)] preparation (Example 14). A yield of 3.82 g of an off-white crystalline material was obtained. NMR data are given in Tables 1 and 2.

## EXAMPLE 20

Na[Pt(trans-R,R-dach)(phosphonoformate)]

The complex, Pt(trans-R,R-dach)$I_2$ (10 mmole) and silver nitrate (20 mmole) were mixed in 200 mL of water at 45°C for one hour and the resulting silver iodide precipitate removed by filtration. To the filtrate

was added 10 mmole of trisodium phosphonoformate. After stirring for 24 hours the solution was evaporated to dryness and the residue stirred in absolute ethanol. The resulting white solid was filtered, washed with absolute ethanol and air dried 48 hours. A portion of the product was recrystalized from acetone/water. NMR data are presented in Tables 1 and 2.

EXAMPLE 21

Na[cis-Pt($NH_3$)$_2$(phosphonoacetate)]

A mixture of 10 mmole cis-Pt($NH_3$)$_2$($SO_4$)($H_2O$),10 mmole Ba(OH)•8$H_2O$ and 11 mmole of phosphonoacetic acid was stirred for two hours at 25°C in 50 mL of water. The barium sulfate precipitate was removed by filtration and the filtrate was reduced to dryness and then suspended in isopropanol. This precipitate was filtered, air dried, and further purified by mixing a portion of the solid in water and adjusting the pH to 8.3 with 6 N NaOH. The small amount of insoluble material that remained was filtered from the solution. The remaining filtrate yielded an off-white precipitate after evaporation. NMR data are presented in Tables 1 and 2.

EXAMPLE 22

Na[Pt(trans-R,R-dach)(2-methyl-2-phosphonobutyrate)]

A mixture of 3.59 mmole Pt(trans-R,R-dach)($SO_4$)($H_2O$), 3.59 mmole $Ba(OH)_2$•8$H_2O$, 3.59 mmole NaOH and 3.59 mmole of 2-methyl-2-phosponobutyric acid in 100 mL of water was stirred at 45°C for 20 hours. The resulting $BaSO_4$ precipitate was removed by filtration and the filtrate was roto-evaporated to dryness. The solid was washed with ethanol and vacuum dried. $^{195}$Pt,$^{13}$C and $^{31}$P NMR spectrum indicate that the complex is the expected product.

EXAMPLE 23

Na[Pt(trans-R,R-dach)(2-phosphono-1,7-heptanedicarboxylate)]

A mixture of 4.164 mmole each of Pt(trans-R,R-dach)($SO_4$)($H_2O$), 2-phosphono-1,7-heptanedicarboxylic acid, $Ba(OH)_2$•8H20 and NaOH was stirred in 100 mL of water at 50°C overnight. The $BaSO_4$ was removed by filtration and the filtrate was rotoevaporated to dryness. The product can be purified by recrystallization from methanol. $^{195}$Pt,$^{13}$C, and $^{31}$P NMR were used to confirm the identity of the product.

TABLE 1

NMR Data for Pt(diamine)(phosphonocarboxylate) Complexes

| Complex[a] | $^{31}P$ $\delta$ (ppm)[b] | $^{195}Pt$ $\delta$ (ppm)[c] |
|---|---|---|
| Na[cis-Pt($NH_3$)$_2$(PAC)](Ex 21) | 24.2 | -1591 |
| Na[cis-Pt($NH_3$)$_2$(PPA)](Ex 15) | 27.6 | -1590 |
| Na[cis-Pt($NH_3$)$_2$(PBA)](Ex 14) | 27.5 | -1585 |
| Na[cis-Pt($NH_3$)$_2$(PVA)](Ex 19) | 27.8 | -1595 |
| Na[cis-Pt($NH_3$)$_2$(PhenPAC)](Ex 7) | 23.4 | -1614 |
| Na[cis-Pt(i-Pr$NH_2$)$_2$(PAC)](Ex 9) | 23.4 | -1717 |
| H[Pt(en)(PFA)](Ex 13) | 9.4 | -1932 |
| Na[Pt(en)(PAC)](Ex 17) | 23.7 | -1852 |
| Na[Pt(1,2-pn)(PAC)](Ex 11) | 23.8 | -1803 |
| Na[Pt(1,2-pn)(PPA)](Ex 12) | 27.1 | -1824 |
| Na[Pt(1,2-pn)(PBA)](Ex 8) | 26.9 | -1803 |
| Na[Pt(R,R-dach)(PFA)](Ex 20) | 10.2 | -1886 |
| Na[Pt(R,R-dach)(PVA)](Ex 18) | 27.3 | -1807 |
| Na[Pt(R,R-dach)(PhenPAC)](Ex 6) | 23.1 | -1818 |
| Na[Pt(R,R-dach)(MPBA)](Ex 22) | 31.5 | -1787 |
| Na[Pt(R,R-dach)(PHD)](Ex 23) | 27.5 | -1806 |

[a] Abbrev. en=ethylenediamine; 1,2-pn=1,2-diaminopropane; dach=1,2-diaminocyclohexane; PFA=phosphonoformate; PAC=phosphonoacetate; PPA=2-phosphonopropionate; PBA=2-phosphonobutyrate; PVA=2-phosphonovalerate; PhenPAC=2-phosphonophenylacetate; MPBA=2-methyl-2-phosphonobutyrate; PHD=2-phosphono-1,7-heptanedicarboxylate; R,R-dach=trans-R,R-dach; i-Pr=isopropyl; Ex=Example

[b] Reference: $H_3PO_4$ at 0 ppm.

[c] Reference: $H_2PtCl_6$(1g/3mLD20) at 0 ppm.

TABLE 2

$^{13}C$ Chemical Shift Data (ppm) For Pt(diamine)(phosphonocarboxylates)

| Complex[a] | Amine C1 | Amine C2 | Amine C3 | Amine C4 | Amine C5 | Amine C6 | Phosphonocarboxylate Ligand C1 | C2 | C3 | C4 | C5 | C6 | C7 | C8 |
|---|---|---|---|---|---|---|---|---|---|---|---|---|---|---|
| [cis-Pt($NH_3$)$_2$(PAC)](E 21) | | | | | | | 182.14<br>182.00 | 41.57<br>39.31 | | | | | | |
| [cis-Pt($NH_3$)$_2$(PPA)](E 15) | | | | | | | 185.14<br>c | 46.07<br>43.79 | 13.03<br>12.96 | | | | | |
| [cis-Pt($NH_3$)$_2$(PBA)](E 14) | | | | | | | 184.63<br>184.55 | 54.21<br>51.93 | 22.03<br>21.95 | 13.69<br>13.41 | | | | |
| [cis-Pt($NH_3$)$_2$(PVA)](E 19) | | | | | | | 184.82<br>184.72 | 52.20<br>49.92 | 30.82<br>30.74 | 22.43<br>22.17 | 13.79 | | | |
| [cis-Pt($NH_3$)$_2$(PhenPAC)](E 7) | | | | | | | 182.92<br>182.82 | 59.10<br>56.86 | 136.94<br>136.81 | 130.23 | 128.87 | 127.27 | 128.97 | 130.12 |
| [cis-Pt(i-Pr$NH_2$)$_2$(PAC)](E 9) | 49.06(48.93) | 23.12(22.97) | | | | | 182.14<br>182.03 | 42.37<br>40.13 | | | | | | |
| Pt(en)(PPA)](E 13) | 50.56 | 50.56 | | | | | 184.20<br>c | | | | | | | |
| [Pt(en)(PAC)](E 17) | 51.25 | 51.07 | | | | | 181.82<br>c | 43.11<br>40.76 | | | | | | |
| Pt(1,2-pn)(PAC)](E 11) | 53.62 | 56.83 | 15.68 | | | | 133.57<br>133.46 | 41.90<br>39.64 | | | | | | |
| [Pt(1,2-pn)(PPA)](E 12) | 53.57(53.47)<br>53.28(53.11) | 56.85(56.54)<br>56.43(56.29) | 15.79(d)<br>c | | | | 184.68<br>184.61 | 46.01<br>43.72 | 12.70<br>12.65 | | | | | |
| [Pt(1,2-pn)(PBA)](E 8) | 53.54(53.2) | 56.71(56.35) | 15.77(d) | | | | 184.50<br>184.21 | 54.40<br>52.12 | 21.99<br>21.92 | 13.73<br>13.45 | | | | |
| [Pt(R,R-dach)(PPA)](E 20) | 63.03 | 62.88 | 32.24 | 24.43 | 24.43 | 32.24 | 186.97<br>182.62 | | | | | | | |
| [Pt(R,R-dach)(PVA)](E 18) | 63.29<br>63.05 | 64.73<br>c | 32.29<br>c | 24.41<br>24.28 | 24.41<br>24.28 | 32.29<br>c | 184.63(184.52)<br>184.37(184.27) | 52.34(52.28)<br>50.08(50.01) | 30.78(30.7)<br>30.61(30.54) | 22.43<br>22.17 | 13.78 | | | |
| [Pt(R,R-dach)(PhenPac)](E 6) | 63.48<br>c | 62.94<br>62.87 | 32.31<br>c | 24.42 | 24.42 | 32.31<br>c | 182.84<br>182.78 | 59.25(59.18)<br>57.02(56.97) | 136.79<br>136.70 | 130.23<br>130.11 | 128.79<br>c | 127.23<br>127.19 | 128.79<br>c | 130.23<br>130.11 |
| [Pt(R,R-dach(PID)](E 23) | 62.66 | 62.92 | 31.92 | 24.07 | 24.07 | 31.92 | 183.81(183.69)<br>183.63(183.54) | 51.95(51.92)<br>49.69(49.66) | 28.42<br>28.15 | 27.89(27.81)<br>27.81(27.71) | 25.48<br>25.43 | 36.53 | 182.51 | |
| [Pt(R,R-dach)(MEBA)](E 22) | 62.48(62.30) | 63.06(62.88) | 31.87 | 23.97 | 23.97 | 31.87 | 186.31(186.23) | 51.48<br>49.09 | 28.48 | 17.34 | 8.69<br>8.45 | | | |

**TABLE 2 Cont.**

**$^{13}C$ Chemical Shift Data (ppm) for Pt(diamine)(phosphonocarboxylates)**

| Ligand[a] | Amine C1 | Amine C2 | Amine C3 | Amine C4 | Amine C5 | Amine C6 | Uncoordinated Phosphonocarboxylate Ligand C1 | C2 | C3 | C4 | C5 | C6 | C7 | C8 |
|---|---|---|---|---|---|---|---|---|---|---|---|---|---|---|
| PVA (E 2) | | | | | | | 174.66<br>174.57 | 48.04<br>45.55 | 29.30<br>29.20 | 21.70<br>21.40 | 13.34<br>c | | | |
| PHD (E 4) | | | | | | | 175.14<br>175.05 | 48.63<br>46.21 | 27.87<br>27.57 | 27.14<br>27.05 | 24.34 | 33.08 | 179.16 | |
| MPBA (E 3) | | | | | | | 175.15<br>175.10 | 48.41<br>45.87 | 25.6 | 14.76 | 6.96(e)<br>6.71(e) | | | |

[a]Abbrev.; en=ethylenediamine; 1,2-pn=1,2-diaminopropane; dach=1,2-diaminocyclohexane; PFA=phosphonoformate; PAC=phosphonoacetate; PPA=2-phosphonopropionate; PBA=2-phosphonobutyrate; PVA=2-phosphonovalerate; PhenPAC=2-phosphonophenylacetate; PHD=2-phosphono-1,7-heptanedicarboxylate; MPBA=2-methyl-2-phosphonobutyrate; i-Pr=isopropyl

E=Example

b $^{31}P$ coupling indicated by vertical double column entries; isomers noted in parentheses

c splitting due to J(P-C) was unresolved

d isomers were unresolved

e $C_5$ is methyl substituent on $C_3$

## TABLE 3

### Elemental Analysis

| Compound[a]/Formula | | % C | % H | % N | % P | % Pt | % Na |
|---|---|---|---|---|---|---|---|
| Na[cis-Pt(i-PrNH$_2$)$_2$PAC]·IPA·$H_2O$ | calc: | 23.96 | 5.48 | 5.08 | 5.62 | 35.38 | 4.17 |
| $C_{11}H_{30}N_2PPtNaO_7$ (Ex 9) | found: | 21.15 | 4.91 | 5.51 | 6.11 | 36.83 | 3.81 |
| Na[Pt(en)PAC]·2$H_2O$ | calc: | 10.65 | 3.13 | 6.21 | 6.86 | 43.24 | 5.1 |
| $C_4H_{14}N_2PPtNaO_7$ (Ex 17) | found: | 11.04 | 3.05 | 6.42 | 7.14 | 41.9 | 4.73 |
| Na[Pt(1,2-pn)PAC]·MeOH | calc: | 15.62 | 3.50 | 6.07 | 6.72 | 42.30 | 4,98 |
| $C_6H_{16}N_2PPtNaO_6$ (Ex 11) | found: | 14.65 | 3.62 | 6.01 | 6.70 | 40.04 | 4.06 |
| Na[Pt(1,2-pn)PBA]·2$H_2O$ | calc: | 17.04 | 4.09 | 5.68 | 6.28 | 39.55 | 4.66 |
| $C_7H_{20}N_2PPtNaO_7$ (Ex 8) | found: | 17.70 | 3.95 | 5.54 | 6.28 | 37.46 | 4.36 |

a Abbreviations en=ethylenediamine; 1,2-pn=1,2-diaminopropane; IPA=isopropanol; MeOH=methanol; PAC=phosphonoacetate; PBA=2-phosphonobutyrate; i-Pr=isopropyl; Ex=Example

EXAMPLE 24

Anti-tumor Evaluation

The platinum-phosphonocarboxylate compounds of the Examples were tested for anti-tumor activity using both the Sarcoma 180 ascites and the Ll210 leukemia tumor models. Each compound was evaluated in at least one of these screens.

Ll210 Screening Methodology

Female $CDF_1$ mice (16-22 g) are injected with a suspension of 1 x $10^6$ tumor cells in 0.5 mL of 0.15 M saline on day zero. On day one, the mice receive the test compound in 0.5 mL of the chosen medium (usually water). Six mice were used for each test dose, with six doses normally being run. Normal controls (six mice) receive tumor on day zero and 0.5 mL of the test medium on day one. Positive controls (six mice) receive tumor on day zero and 8 mg/kg cisplatin in 0.5 mL of 0.15 M saline on day one. All compound doses are adjusted for the actual average weight of the mice involved. Testing is terminated at three times the normal control mean life span. The percent increase in lifespan (ILS) is calculated as follows:

%ILS = [(mean life span (test)/mean life span(control)-l]x100

A 25% ILS or greater indicates activity. The maximum possible ILS is 200%. The positive control must have an ILS of ≥ 25% for the test to be considered valid.

The tumor line is maintained by transfer of 5 x $10^4$ cells in 0.1 mL saline every seven days. DBA/2 (18-22 g) mice are employed for tumor transfers

Sl80a Screening Methodology

CFW mice, averaging 20 g, are innoculated on day zero with 0.2 mL of a freshly prepared saline suspension (0.15 M NaCl) containing 1 x $10^7$ tumor cells/mL, or a total of 2 x $10^6$ cells. On day l, the mice are injected with the test compound in the selected medium (usually water). Six mice are used per test dose, with six doses normally being run.

Also, on day one, two types of controls (6 mice/set are employed: (1) Normal (1 set): 0.5 mL of the solvent medium used for the test compound, and (2) Positive control (1 set): cis-$[Pt(NH_3)_2Cl_2]$ in saline at 8 mg/kg.

The effectiveness of a compound is measured in terms of the increase in life span (ILS) of the test animals relative to the controls calculated from the day of tumor inoculation (day zero). The day of evaluation is arbitrarily taken as that day corresponding to twice the mean life-span of the normal controls. This sets a practical upper limit of 100% on the ILS attainable. For calculation purposes, survivors on the day of evaluation are considered to have died on that day. The % ILS is formulated as:

$$ILS = \left(\frac{\text{mean life-span of test mice}}{\text{mean life-span of control mice}} - 1\right) \times 100\%$$

ILS values above 50% represent activity. The positive controls must have an ILS of ≥ 50% for the test to be valid. The test results are shown in Tables 4 and 5.

TABLE 4

S180a Screening Data For Pt(diamine)(phosphonocarboxylate) Complexes

| Compound (Vehicle) | DOSE mg/kg | %ILS | Surv. | POS. CONT. %ILS | POS. CONT. Surv. |
|---|---|---|---|---|---|
| H[Pt(en)(phosphonoacetate)] (water) (Ex16) | 5.00 | -2 | 0/ 6 | 94 | 4/ 6 |
| | 10.00 | 3 | 0/ 6 | | |
| | 20.00 | 29 | 1/ 6 | | |
| | 40.00 | 64 | 3/ 6 | | |
| | 80.00 | 84 | 4/ 6 | | |
| | 160.00 | 85 | 3/ 6 | | |
| H[Pt(en)(phosphonoacetate)] (water, pH7) (Ex 16) | 5.00 | -3 | 0/ 6 | 89 | 4/ 6 |
| | 10.00 | 2 | 0/ 6 | | |
| | 20.00 | 14 | 0/ 6 | | |
| | 40.00 | 72 | 2/ 6 | | |
| | 80.00 | 93 | 4/ 6 | | |
| | 160.00 | -32 | 1/ 6 | | |
| H[Pt(en)(phosphonoformate)] (water, pH7) (Ex 13) | 5.00 | 9 | 0/ 6 | 89 | 4/ 6 |
| | 10.00 | 31 | 1/ 6 | | |
| | 20.00 | 63 | 1/ 6 | | |
| | 40.00 | 93 | 5/ 6 | | |
| | 80.00 | 58 | 2/ 6 | | |
| | 160.00 | -74 | 0/ 6 | | |
| Na[Pt(dach)(phosphonoformate)] (water) (Ex 20) | 10.00 | 6 | 0/ 6 | 85 | 3/ 6 |
| | 20.00 | -5 | 0/ 6 | | |
| | 40.00 | 7 | 0/ 6 | | |
| | 80.00 | 18 | 0/ 6 | | |
| | 160.00 | 59 | 0/ 6 | | |
| | 320.00 | 55 | 0/ 6 | | |
| H[Pt(1,2-pn)2-phosphonoacetate)] (water) (Ex 10) | 20.00 | 77 | 3/ 6 | 70 | 2/ 6 |
| | 40.00 | 9 | 1/ 6 | | |
| | 80.00 | -71 | 0/ 6 | | |
| | 160.00 | -78 | 0/ 6 | | |
| | 320.00 | -79 | 0/ 6 | | |
| Na[cis-Pt($NH_3$)$_2$(2-phosphonopropionate)] (water) (Ex 15) | 10.00 | 16 | 1/ 6 | 55 | 1/ 6 |
| | 20.00 | 1 | 0/ 6 | | |
| | 40.00 | -7 | 0/ 6 | | |
| | 80.00 | -80 | 0/ 6 | | |
| | 160.00 | -80 | 0/ 6 | | |

TABLE 4 Cont.

S180a Screening Data For Pt(diamine)(phosphonocarboxylate)

| Compound (Vehicle) | DOSE mg/kg | %ILS | Surv. | POS. CONT. %ILS | POS. CONT. Surv. |
|---|---|---|---|---|---|
| Na[Pt(1,2-pn)(2-phosphono-butyrate)] (water) (Ex 8) | 5.00 | 4 | 0/ 6 | 89 | 3/ 6 |
| | 10.00 | 19 | 0/ 6 | | |
| | 20.00 | 4 | 0/ 6 | | |
| | 40.00 | 46 | 0/ 6 | | |
| | 80.00 | 38 | 0/ 6 | | |
| | 160.00 | 95 | 4/ 6 | | |
| Na[Pt(1,2-pn)(2-phosphono-propionate)] (water) (Ex 12) | 20.00 | 20 | 0/ 6 | 74 | 2/ 6 |
| | 40.00 | 24 | 0/ 6 | | |
| | 80.00 | 40 | 0/ 6 | | |
| | 160.00 | 53 | 1/ 6 | | |
| Na[cis-Pt($NH_3$)$_2$(2-phosphono-valerate)] (water) (Ex 19) | 20.00 | -74 | 0/ 6 | 74 | 2/ 6 |
| | 40.00 | -85 | 0/ 6 | | |
| | 80.00 | -95 | 0/ 6 | | |
| | 160.00 | -95 | 0/ 6 | | |
| Na[Pt(R,R-dach)(2-phosphono-valerate)] (water) (Ex 18) | 40.00 | 77 | 1/ 6 | 86 | 3/ 6 |
| | 80.00 | 92 | 5/ 6 | | |
| | 160.00 | 44 | 1/ 6 | | |
| Na[cis-Pt($NH_3$)$_2$(2-phosphono-phenylacetate)] (water) (Ex 7) | 20.00 | 14 | 0/ 6 | 86 | 3/ 6 |
| | 40.00 | 26 | 0/ 6 | | |
| | 80.00 | 71 | 2/ 6 | | |
| | 160.00 | 28 | 0/ 6 | | |
| Na[Pt(R,R-dach)(2-phosphono-phenylacetate)] (water) (Ex 6) | 40.00 | 24 | 0/ 6 | 74 | 2/ 6 |
| | 80.00 | 88 | 2/ 6 | | |
| | 160.00 | 102 | 5/ 6 | | |
| Na[cis-Pt(i-PrNH$_2$)$_2$(2-phos-phonoacetate)] (water) (Ex 9) | 20.00 | 1 | 0/ 6 | 70 | 2/ 6 |
| | 40.00 | -2 | 0/ 6 | | |
| | 80.00 | -4 | 0/ 6 | | |
| | 160.00 | 15 | 0/ 6 | | |
| | 320.00 | 17 | 0/ 6 | | |
| Na[Pt(R,R-dach)(2-methyl-2-phosphonobutyrate)] (water) (Ex 22) | 10.00 | 28 | 1/ 6 | 68 | 3/ 6 |
| | 20.00 | 20 | 0/ 6 | | |
| | 40.00 | 82 | 4/ 6 | | |
| | 80.00 | 100 | 6/ 6 | | |
| | 160.00 | 86 | 2/ 6 | | |
| | 320.00 | -78 | 0/ 6 | | |

TABLE 4 Cont.

S180a Screening Data For Pt(diamine)(phosphonocarboxylate)

| Compound (Vehicle) | DOSE mg/kg | %ILS | Surv. | POS. CONT. %ILS | POS. CONT. Surv. |
|---|---|---|---|---|---|
| Na[Pt(R,R-dach)(2-phosphono-1,7-heptanedicarboxylate)] (water) (Ex 23) | 10.00 | 20 | 0/ 6 | 68 | 3/ 6 |
| | 20.00 | 31 | 0/ 6 | | |
| | 40.00 | 45 | 2/ 6 | | |
| | 80.00 | 86 | 4/ 6 | | |
| | 160.00 | 34 | 4/ 6 | | |
| | 320.00 | -37 | 1/ 6 | | |

TABLE 5

L1210 Screening Data For Pt(diamine)(phosphonocarboxylate) Complexes

| Compound (Vehicle) | DOSE mg/kg | %ILS | Surv. | POS. CONT. %ILS | POS. CONT. Surv. |
|---|---|---|---|---|---|
| Na[Pt(trans-R,R-dach)(phosphonoformate)] (water) (Ex 20) | 10.00 | -17 | 0/ 6 | 76 | 1/ 6 |
| | 20.00 | -20 | 0/ 6 | | |
| | 40.00 | -14 | 0/ 6 | | |
| | 80.00 | -7 | 0/ 6 | | |
| | 160.00 | 11 | 0/ 6 | | |
| | 320.00 | 20 | 0/ 6 | | |
| Na[cis-Pt($NH_3$)$_2$(phosphonoacetate)] (water) (Ex 21) | 10.00 | -10 | 0/ 6 | 38 | 0/ 6 |
| | 20.00 | -5 | 0/ 6 | | |
| | 40.00 | 13 | 0/ 6 | | |
| | 80.00 | 12 | 0/ 6 | | |
| | 160.00 | 10 | 0/ 6 | | |
| | 320.00 | -80 | 0/ 6 | | |
| H[Pt(1,2-pn)(phosphonoacetate)] (water) (Ex 10) | 5.00 | -5 | 0/ 6 | 55 | 0/ 6 |
| | 10.00 | 2 | 0/ 6 | | |
| | 20.00 | -13 | 0/ 6 | | |
| | 40.00 | -13 | 0/ 6 | | |
| | 80.00 | -34 | 0/ 6 | | |
| | 160.00 | -52 | 0/ 6 | | |
| Na[cis-Pt($NH_3$)$_2$(2-phosphonobutyrate)] (water) (Ex 14) | 10.00 | -10 | 0/ 6 | 86 | 1/ 6 |
| | 20.00 | -8 | 0/ 6 | | |
| | 40.00 | -15 | 0/ 6 | | |
| | 80.00 | -5 | 0/ 6 | | |
| | 160.00 | -67 | 0/ 6 | | |
| | 320.00 | -80 | 0/ 6 | | |
| Na[Pt(trans-R,R-dach)(2-phosphonovalerate)] (water) (Ex 18) | 10.00 | 36 | 0/ 6 | 86 | 1/ 6 |
| | 20.00 | 25 | 0/ 6 | | |
| | 40.00 | 33 | 0/ 6 | | |
| | 80.00 | 33 | 0/ 6 | | |
| | 160.00 | 109 | 0/ 6 | | |
| | 320.00 | 24 | 1/ 6 | | |
| Na[Pt(trans-R,R-dach)(2-phosphonophenylacetate)] (water) (Ex 6) | 5.00 | 2 | 0/ 6 | 68 | 0/ 6 |
| | 10.00 | 6 | 0/ 6 | | |
| | 20.00 | 26 | 0/ 6 | | |
| | 40.00 | 47 | 0/ 6 | | |
| | 80.00 | 53 | 0/ 6 | | |
| | 160.00 | 76 | 0/ 6 | | |

TABLE 5 Cont.

L1210 Screening Data For Pt(diamine)(phosphonocarboxylate) Complexes

| Compound (Vehicle) | DOSE mg/kg | %ILS | Surv. | POS. CONT. %ILS | POS. CONT. Surv. |
|---|---|---|---|---|---|
| Na[Pt(1,2-pn)(2-phosphono-butyrate)] (water) (Ex 8) | 10.00 | -6 | 0/ 6 | 130 | 3/ 6 |
| | 20.00 | -9 | 0/ 6 | | |
| | 40.00 | 0 | 0/ 6 | | |
| | 80.00 | -2 | 0/ 6 | | |
| | 160.00 | 16 | 0/ 6 | | |
| | 320.00 | -44 | 0/ 6 | | |
| Na[Pt(R,R-dach)(2-methyl-2-phosphonobutyrate)] (water) (Ex 22) | 10.00 | 45 | 0/ 6 | 119 | 2/ 6 |
| | 20.00 | 35 | 0/ 6 | | |
| | 40.00 | 70 | 1/ 6 | | |
| | 80.00 | 94 | 1/ 6 | | |
| | 160.00 | 114 | 3/ 6 | | |
| | 320.00 | -39 | 0/ 6 | | |
| Na[Pt(R,R-dach)(2-phosphono-1,7-heptanedicarboxylate)] (water) (Ex 23) | 10.00 | 59 | 0/ 6 | 74 | 1/ 6 |
| | 20.00 | 67 | 0/ 6 | | |
| | 40.00 | 88 | 0/ 6 | | |
| | 80.00 | 133 | 3/ 6 | | |
| | 160.00 | 161 | 3/ 6 | | |
| | 320.00 | -32 | 0/ 6 | | |

## EXAMPLE 25

### Toxicity Testing

Toxicity testing was performed on one of the Pt(diamine)(phosphonocarboxylate) analogs that showed good antitumor activity in the Ll210 screen: Na[Pt(trans-R,R-dach)(PVA)] (PVA = 2-phosphonovalerate). Based on the screening results, initial dosing was established at approximately twice the peak active dose in the Sl80 screen. Both a cisplatin control, also run at twice the peak active dose, and a negative control experiment, were run for comparative purposes. BUN was measured by using a kit manufactured by Harleco (No. 64667). WBC was measured by lysing the red blood cells with 3% acetic acid and counting the white cells using a haemocytometer.

Data obtained for Na[Pt(trans-R,R-dach)(PVA)] was taken at 240 mg/kg. The average BUN level for the test mice was 34.7 mg/dL, a 4% decrease vs. controls (36.0 mg/dL). The lack of BUN elevation indicates that this compound is not kidney toxic at this dose. Myelosuppresion however, was apparent. The WBC depression for this compound was 63% relative to controls. In addition, low hematocrits (ratio of packed red blood cells to volume of whole blood), were also seen. Average cage weights for the test mice were also taken over the seven day test period. The mice lost an average of 5.9 g (27.3%) of body weight over this time. The BUN and WBC test results are summarized in Table 6.

TABLE 6

Toxicity Testing

| COMPOUND | DOSE | BUN | WBC |
|---|---|---|---|
| Cisplatin | 15 mg/kg | +98% | -7% |
| Pt(R,R-dach)(PVA) | 240 mg/kg | -4% | -63% |

**Claims**

1: A compound of the formula (I):

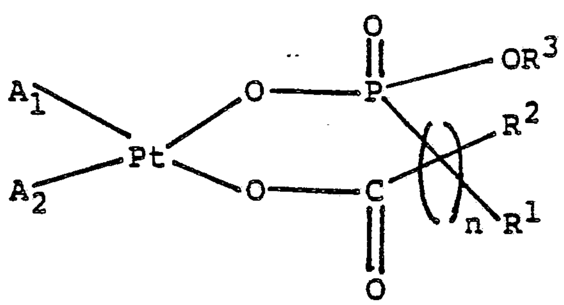

(I)

wherein $A_1$ and $A_2$ are the same or different monodentate amine ligands represented by the formula: $RNH_2$, wherein R is selected from the group consisting of H, $C_1$-$C_6$ alkyl and a substituted $C_1$-$C_6$ alkyl wherein the alkyl may be substituted by any one of hydroxy, $C_1$-$C_4$ alkoxy, halo, carboxy, $C_1$-$C_4$ alkylcarboxy or $C_1$-$C_4$ alkoxycarbonyl, or $A_1$ and $A_2$, taken together, are chelating diamine ligands represented by the formula (II):

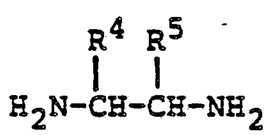

(II)

wherein each of $R^4$ and $R^5$, taken separately, are any of hydrogen, $C_1$-$C_3$ linear or branched alkyl, hydroxy, $C_1$-$C_4$ alkoxy, halo, carboxy, $C_1$-$C_4$ alkylcarboxy, $C_1$-$C_4$ alkoxycarbonyl or linear or branched substituted alkyl, substituted with any of hydroxy, $C_1$-$C_4$ alkoxy, halo, carboxy, $C_1$-$C_4$ alkylcarboxy, $C_1$-$C_4$ alkoxycarbonyl, or wherein $R^4$ and $R^5$, taken together, are any of cycloalkyl containing from about 4-8 nuclear carbon atoms or cycloalkyl substituted at any of the nuclear carbon atoms by at least one linear or branched $C_1$-$C_3$ alkyl, hydroxy, $C_1$-$C_4$ alkoxy, halo, carboxy, $C_1$-$C_4$ alkylcarboxy or $C_1$-$C_4$ alkoxycarbonyl;

n = 0-2;

wherein, when n = 1 or 2, each of the ligands may be substituted by $R^1$ and $R^2$;

$R^1$ and $R^2$ are the same or different and are selected from the group consisting of hydrogen, OH, linear or branched $C_1$-$C_{16}$ alkyl, linear or branched $C_1$-$C_{16}$ alkyl substituted by a substituent functional group, linear or branched $C_1$-$C_{16}$ alkyl ether, linear or branched $C_1$-$C_{16}$ alkyl ether substituted by a substituent functional group, linear or branched $C_1$-$C_{16}$ primary or secondary alkylamine, a linear or branched $C_1$-$C_{16}$

primary or secondary alkyl amine substituted with the substituent functional group, $C_1$-$C_{16}$ linear or branched alkenyl, $C_1$-$C_{16}$ linear or branched alkenyl substituted with the substituent functional group, phenyl, phenyl substituted with the substituent functional group, $C_1$-$C_{16}$ alkyl, $C_1$-$C_{16}$ alkenyl, the $C_1$-$C_{16}$ substituted linear or branched substituted alkyl or the $C_1$-$C_{16}$ substituted linear or branched substituted alkenyl, benzyl and benzyl substituted with the substituent functional groups, $C_1$-$C_{16}$ alkyl, $C_1$-$C_{16}$ alkenyl, the substituted $C_1$-$C_{16}$ alkyl or the substituted $C_1$-$C_{16}$ alkenyl; the substituent functional group is selected from the group consisting of hydroxyl, halo, carboxylic acid, carboxylic acid halide, carboxylic acid hydrazide, ester, primary or secondary amide, carboxylic acid anhydride, aldehyde, acetal, hemiacetal, ketone, ketal, -O-acylurea, imidazole, methoxymethyl ester, tetrahydropyranyl ester, a sulfonic acid ester, hydrazine, hydrazone, semicarbazone, phenyl or the substituted phenyl; and wherein $R^3$ is an alkali metal cation, $H^+$ or $C_1$-$C_4$ alkyl.

2: A compound of the formula I

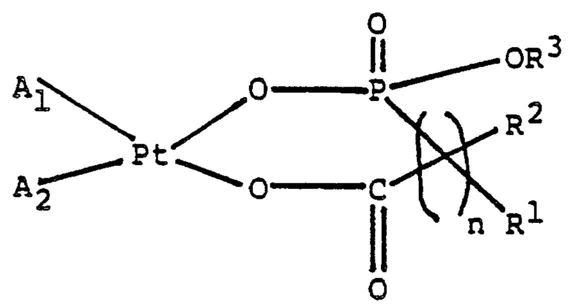

(I)

wherein $A_1$ and $A_2$ are the same or different monodentate amine ligands represented by the formula: $RNH_2$, wherein R is selected from the group consisting of H, $C_1$-$C_6$ alkyl and a substituted $C_1$-$C_6$ alkyl, or $A_1$ and $A_2$, taken together, are chelating diamine ligands represented by the formula (II):

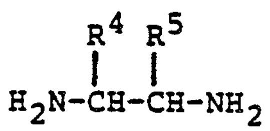

(II)

wherein each of $R^4$ and $R^5$, taken separately, are any of hydrogen, $C_1$-$C_3$ linear or branched alkyl, hydroxy, $C_1$-$C_4$ alkyl, halo, carboxy, $C_1$-$C_4$ alkylcarboxy, $C_1$-$C_4$ alkoxycarbonyl or linear or branched substituted alkyl, or wherein $R^4$ and $R^5$, taken together, are any of cycloalkyl containing from about 4-8 nuclear carbon atoms or cycloalkyl substituted at any of the nuclear carbon atoms;

$n = 0$-$2$;

wherein, when $n = 1$ or 2, each of the ligands may be substituted by $R^1$ and $R^2$;

$R^1$ and $R^2$ are the same or different and are selected from the group consisting of hydrogen, OH, linear or branched $C_1$-$C_{16}$ alkyl, linear or branched $C_1$-$C_{16}$ substituted alkyl, linear or branched $C_1$-$C_{16}$ alkyl ether, linear or branched $C_1$-$C_{16}$ primary or secondary alkylamine, linear or branched $C_1$-$C_{16}$ primary or secondary substituted alkyl amine, $C_1$-$C_{16}$ linear or branched alkenyl, $C_1$-$C_{16}$ linear or branched substituted alkenyl, phenyl, substituted phenyl, benzyl and substituted benzyl; and

wherein $R^3$ is an alkali metal cation, $H^+$ or $C_1$-$C_4$ alkyl.

3: The compound of claim I, wherein $A_1$ and $A_2$ are monodentate amine ligands and R is H or $C_1$-$C_3$ alkyl.

4: The compound of claim I, wherein $A_1$ and $A_2$ are taken together and are bidentate diamine ligands and wherein at least one of $R^4$ and $R^5$ are hydrogen.

5: The compound of claim I, wherein $A_1$ and $A_2$ are taken together and $R^4$ and $R^5$, taken together, afford a 1,2-diaminocyclohexane ligand.

6: The compound of claim I, wherein the $R^3$ alkali metal cations are selected from the group consisting of $Li^+$, $Na^+$ and $K^+$.

7: The compound of claim I, wherein $n = 0$.

8: The compound of claim I, wherein $n = 1$.

9: The compound of claim I, wherein $n = 1$ and $R^1$ and $R^2$ are hydrogen.

10: The compound of claim 1, wherein $R^1$ is phenyl and $R^2$ is H.

11: The compound of claim 1, wherein $R^1$ is $C_1$-$C_{16}$ linear or branched alkyl and $R^2$ is H.

12: The compound of claim 1, wherein $R^1$ and $R^2$ are $C_1$-$C_{16}$ linear or branched alkyl.

13: The compound of claim 1, wherein $R^1$ is $C_1$-$C_{16}$ linear or branched alkyl substituted with a carboxylic acid substituent and $R^2$ is a linear or branched alkyl.

14: The compound of claim 1, wherein $R^4$ and $R^5$ are taken separately.

15: The compound of claim 1, wherein $R^4$ and $R^5$ are taken together.

16: The compound of claim 1, selected from the following:

Na[Pt(trans-R,R-dach)(2-phosphonophenylacetate)]
Na[cis-Pt($NH_3$)$_2$(2-phosphonophenylacetate)]
Na[Pt(diaminopropane)(2-phosphonobutyrate)]
Na[cis-Pt(isopropylamine)$_2$(phosphonoacetate)]
H[Pt(diaminopropane)(phosphonoacetate)]
Na[Pt(diaminopropane)(phosphonoacetate)]
Na[Pt(diaminopropane)(2-phosphonopropionate)]
H[Pt(ethylenediamine)(phosphonoformate)]
Na[cis-Pt($NH_3$)$_2$(2-phosphonobutyrate)]
Na[cis-Pt($NH_3$)$_2$(2-phosphonopropionate)]
H[Pt(ethylenediamine)(phosphonoacetate)]
Na[Pt(ethylenediamine)(phosphonoacetate)]
Na[Pt(trans-R,R-dach)(2-phosphonovalerate)]
Na[cis-Pt($NH_3$)$_2$(2-phosphonovalerate)]
Na[Pt(trans-R,R-dach)(phosphonoformate)]
Na[cis-Pt($NH_3$)$_2$(2-phosphonoacetate)]
Na[Pt(trans-R,R-dach)(2-methyl-2-phosphonobutyrate)]
Na[Pt(trans-R,R-dach)(2-phosphono-1,7-heptane-dicarboxylate)l

17. A composition comprising as the active ingredient a therapeutically effective amount of a compound according to any preceding Claim in combination with a non-toxic pharmacologically acceptable inert carrier or diluent

18. The composition of claim 17 in a form suitable for parenteral administration.

19. The composition of claim 17 in a form suitable for oral administration.

20. The composition of claim 17 in tablet form

21. The composition of claim 17 in capsule form.

22. The composition of claim 17, wherein the active ingredient is present in an amount of about 5 to 375 mg.

23. The composition of claim 17, wherein the active ingredient is present in an amount of about 10 to 200 mg.

24. A conjugate comprising a compound according to any of claims 1 to 6 wherein n=l or 2, and a monoclonal antibody linked thereto through either the $R^1$ or the $R^2$ substituent.

25. A compound according to any of claims 1 to 16, a composition according to any of claims 17 to 23 or a conjugate according to claim 24 for use in the treatment of the human or animal body by therapy.

26. The use of a compound according to any of claims 1 to 16 in the manufacture of a medicament for the treatment of malignant animal tumors.